# EUROPÄISCHE PATENTSCHRIFT

(11) **EP 1 711 364 B1**
(45) Veröffentlichungstag und Bekanntmachung des Hinweises auf die Patenterteilung: **15.07.2009**
(21) Anmeldenummer: 05701135.5
(22) Anmeldetag: 24.01.2005
(51) Int. Cl.: B60K 28/06

(54) **VORRICHTUNG ZUR ERFASSUNG DER FAHRT CHTIGKEIT EINES FAHRERS IN EINEM FAHRZEUG**
DEVICE FOR RECORDING THE DRIVING CAPACITY OF A DRIVER IN A VEHICLE
DISPOSITIF POUR DETECTER LA CAPACITE DE CONDUIRE D'UN CONDUCTEUR DANS UN VEHICULE

(30) Priorität: 02.02.2004 DE 102004005163
(43) Veröffentlichungstag der Anmeldung: 18.10.2006
(73) Patentinhaber: uwe braun GmbH, 19309 Lenzen (Elbe) (DE)
(72) Erfinder: BRAUN, Uwe Peter Dipl.-Ing., 14467 Potsdam (DE)
(74) Vertreter: Cohausz & Florack
(86) Internationale Anmeldenummer: PCT/EP2005/000639
(87) Internationale Veröffentlichungsnummer: WO 2005/073013

(56) Entgegenhaltungen:
- DE-A1- 3 803 916
- DE-A1- 19 621 435
- DE-C1- 19 803 158
- US-A- 5 422 690

## Beschreibung

Die Erfindung betrifft eine Vorrichtung zur Erfassung der Fahrtüchtigkeit eines Fahrers in einem Fahrzeug, mit einer Beleuchtungseinrichtung zum Beleuchten wenigstens eines Auges des Fahrers, einer Bildaufnahmeeinrichtung zum Aufnehmen von Bildern des beleuchteten Auges, einer Auswerteeinrichtung, die dem Auswerten der von der Bildaufnahmeeinrichtung aufgenommenen Bilder dient, und einem Datenspeicher.

Jährlich kommt es in Verbindung mit Kraftfahrzeugen zu einer Vielzahl von Verkehrsunfällen durch Fahrer, die am Lenkrad einschlafen. Es wurden deshalb bereits verschiedene Vorrichtungen vorgeschlagen, die dazu dienen, den Wachheits-bzw. Vigilanzzustand eines Fahrzeugführers kontinuierlich zu überwachen und ihn während des Fahrens rechtzeitig vor dem Einschlafen zu warnen. Dazu werden von einem Bildaufnahmesystem Bilder des Bereiches wenigstens eines Auges aufgenommen und von einem nachgeschalteten Bildauswertesystem analysiert. Bei Vorrichtungen dieser Art, wie sie z.B. aus der DE 198 03 158 C1 und der DE 196 21 435 A1 bekannt sind, beinhaltet das Bildauswertesystem Mittel zur Lidschlusserkennung, mit denen ein zunehmendes zeitweises Schließen der Augen festgestellt werden kann, was als zunehmende Müdigkeit interpretiert wird. Dem Bildauswertesystem ist eine Warnsystemeinrichtung zugeordnet, die in Abhängigkeit eines festgestellten Müdigkeitszustandes ein Warnsignal für den Fahrzeugführer erzeugt. Die Vorrichtung gemäß DE 198 03 158 C1 weist neben Mitteln zur Lidschlusserkennung auch Mittel zur Pupillendurchmesserbestimmung bzw. zur Erfassung von Pupillendurchmesserschwingungen auf. Die US 5 422 690 stellt den Stand der Technik gemäss der Präambel des Anspruchs 1 dar.

Die Sicherheit im Straßenverkehr wird jedoch nicht nur durch eine Übermüdung bzw. Schläfrigkeit von Fahrern beeinträchtigt; eine hohe Gefahr geht insbesondere auch von Fahrern aus, die unter Einwirkung von Alkohol, Betäubungsmitteln oder Drogen ein Kraftfahrzeug lenken. Allein in Deutschland werden jährlich ca. 40.000 Verkehrsunfälle durch Alkohol am Steuer verursacht, ca. 2.000 davon mit tödlichem Ausgang.

Der vorliegenden Erfindung liegt die Aufgabe zugrunde, in einem Fahrzeug eine Vorrichtung zur Erfassung der Fahrtüchtigkeit eines Fahrers zu schaffen, die geeignet ist, eine Inbetriebnahme bzw. ein Lenken des Fahrzeuges durch einen aufgrund des Konsums von Alkohol oder Drogen oder der Einnahme eines Betäubungsmittels fahruntüchtigen Fahrer zu verhindern.

Diese Aufgabe wird erfindungsgemäß durch eine Vorrichtung mit den Merkmalen des Anspruchs 1 gelöst. Bevorzugte und vorteilhafte Ausgestaltungen der erfindungsgemäßen Vorrichtung sind in den Unteransprüchen angegeben.

Die erfindungsgemäße Vorrichtung umfasst eine Beleuchtungseinrichtung zum Beleuchten wenigstens eines Auges des Fahrers, eine Bildaufnahmeeinrichtung zum Aufnehmen von Bildern des beleuchteten Auges, eine Auswerteeinrichtung, die dem Auswerten der von der Bildaufnahmeeinrichtung aufgenommenen Bilder dient, und einen Datenspeicher. Die Beleuchtungseinrichtung beleuchtet blitzlichtartig oder kurzzeitig intermittierend wenigstens ein Auge des Fahrers, wobei die Auswerteeinrichtung mittels der Bildaufnahmeeinrichtung erfasste Messwerte der Pupillenreaktion des Fahrers mit mindestens einem im Datenspeicher gespeicherten Normalwert einer Pupillenreaktion vergleicht und bei einer Unterschreitung des Normalwertes durch die Messwerte der Pupillenreaktion auf eine Steuerungseinrichtung derart einwirkt, dass eine Inbetriebnahme des Fahrzeuges oder gegebenenfalls nach einem Halt des im Betriebszustand befindlichen Fahrzeuges eine Weiterfahrt verhindert wird.

Damit die erfindungsgemäße Vorrichtung von einem durch Alkohol- oder Drogenkonsum fahruntüchtigen Fahrer nicht überlistet werden kann, sind in dem Datenspeicher auch biometrische Daten einer oder mehrerer Personen bezüglich Irisstruktur, Augenfarbe, Augenabstand, Augenfläche, Nasengröße, Mundgröße und/oder Gesichtsform speicherbar und mittels der Bildaufnahmeeinrichtung entsprechende biometrische Daten des jeweiligen Fahrers erfassbar, wobei die Auswerteeinrichtung zur Identifikation des Fahrers die erfassten biometrischen Daten mit den gespeicherten biometrischen Daten vergleicht und bei Nichtübereinstimmung der verglichenen Daten innerhalb vorgegebener Toleranzgrenzen auf mindestens eine Steuerungseinrichtung derart einwirkt, dass eine Inbetriebnahme des Fahrzeugs oder eine Weiterfahrt nach einem Halt des im Betriebszustand befindlichen Fahrzeugs verhindert wird.

Eine Überlistung der erfindungsgemäßen Vorrichtung in der Weise, dass zunächst eine fahrtüchtige Person das Kraftfahrzeug in Betrieb nimmt und anschließend bei laufendem Motor ein durch Alkohol- oder Drogenkonsum fahruntüchtiger Fahrer die Lenkung und Bedienung des Kraftfahrzeuges übernimmt, lässt sich somit ausschließen.

Zusätzlich oder alternativ hierzu kann die erfindungsgemäße Vorrichtung auch in der Weise ausgestaltet werden, dass in dem Datenspeicher die biometrische Daten mindestens eines Fingerabdruckes speicherbar und mittels eines Sensors biometrische Daten eines Fingerabdruckes des jeweiligen Fahrers erfassbar sind, wobei die Auswerteeinrichtung zur Identifikation des Fahrers wiederum die erfassten biometrischen Daten mit den gespeicherten biometrischen Daten vergleicht und bei Nichtübereinstimmung der verglichenen Daten innerhalb vorgegebener Toleranzgrenzen auf mindestens eine Steuerungseinrichtung derart einwirkt, dass eine Inbetriebnahme des Fahrzeugs oder eine Weiterfahrt nach einem Halt des im Betriebszustand befindlichen Fahrzeugs verhindert wird.

Menschen können ihre Pupillenweite bzw. das Verhalten ihrer Pupillen grundsätzlich nicht willentlich beeinflussen. Die Aufzeichnung und Analyse des spontanen Pupillenverhaltens im Dunkeln über mehrere Minuten gilt als ein objektives Verfahren, das Auskunft gibt über den Grad der unbewusst kontrollierten, zentralnervösen Aktivierung. Eine stabile Pupillenweite entspricht einem hohen Aktivierungsniveau, d.h. einem wachen Zustand, während Pupillenschwingungen ein Zeichen für Schläfrigkeit sind. Pupillographische Untersuchungen an Drogenkonsumenten haben gezeigt, dass bereits bei niedrigen Drogen-Konzentrationen im Blut deutliche Ausfälle hinsichtlich der Pupillenreaktion auf Licht auftreten, und zwar in Form einer trägen Pupillenreaktion oder dem völligen Ausbleiben einer Pupillenreaktion.

Mit der Beleuchtungseinrichtung der erfindungsgemäßen Vorrichtung wird wenigstens ein Auge des Fahrers blitzlichtartig oder kurzzeitig intermittierend beleuchtet und die Pupillenreaktion mittels der Bildaufnahmeeinrichtung erfasst. Die so erfassten Messwerte werden in der Auswerteeinrichtung mit mindestens einem gespeicherten Normalwert bzw. einer Bandbreite zulässiger Normalwerte verglichen. Ergibt der Vergleich eine unzulässige Abweichung der erfassten Messwerte von bei Fahrtauglichkeit üblichen Normalwerten der Pupillenreaktion, so wird eine Inbetriebnahme des Fahrzeuges oder eine Weiterfahrt nach einem Halt des im Betriebszustand befindlichen Fahrzeuges über die Steuerungseinrichtung der erfindungsgemäßen Vorrichtung verhindert.

Da im Falle einer fehlenden oder trägen Pupillenreaktion eine plötzliche Außerbetriebnahme des Kraftfahrzeuges während der Fahrt problematisch ist, verhindert die erfindungsgemäße Vorrichtung vorzugsweise nur bei einem Stillstand des Kraftfahrzeuges dessen Inbetriebnahme bzw. dessen Weiterfahrt, sofern eine unzulässige Abweichung der gemessenen Pupillenreaktion von den bei Fahrtauglichkeit üblichen Normalwerten vorliegt.

Die Option, wonach gegebenenfalls die Weiterfahrt nach einem Halt des im Betriebszustand befindlichen Kraftfahrzeuges verhindert wird, ist insbesondere für den Fall zweckmäßig, in welchem das Kraftfahrzeug zunächst von einer fahrtüchtigen Person in Betrieb genommen wird und anschließend bei laufendem Motor ein Fahrerwechsel mit einem aufgrund von Alkohol- oder Drogenkonsum fahruntüchtigen Fahrer stattfinden soll.

Stellt die erfindungsgemäße Vorrichtung eine fehlende oder träge Pupillenreaktion und damit eine Fahruntüchtigkeit des Fahrers fest, so sperrt die Steuerungseinrichtung der Vorrichtung vorzugsweise die Freigabe des Motorstarts. Alternativ oder zusätzlich kann die Steuerungseinrichtung dann gegebenenfalls auch das Einlegen zumindest von Vorwärtsgängen des Schalt- oder Automatikgetriebes des Fahrzeuges sperren. Letzteres bietet sich insbesondere bei elektrisch schaltbaren Getrieben an.

Eine bevorzugte Ausgestaltung der erfindungsgemäßen Vorrichtung besteht ferner darin, dass die Auswerteeinrichtung bei einer Unterschreitung des gespeicherten Normalwertes einer durch Licht ausgelösten Pupillenreaktion durch die erfassten Messwerte der Pupillenreaktion einen Signalgeber ansteuert, der akustische und/oder optische Warnsignale abgibt. Diese Ausgestaltung ist zweckmäßig, um gegebenenfalls auch während der Fahrt eine Fahruntüchtigkeit des Fahrers zu signalisieren. Als Warnsignale kommen beispielsweise Hubsignale, Sprachsignale und/oder mit den Fahrzeugscheinwerfern und/oder der Warnblinkanlage abgegebene Lichtsignale in Frage. So können insbesondere auch Mitfahrer und andere Verkehrsteilnehmer auf die eventuelle Fahruntüchtigkeit eines Fahrers aufmerksam gemacht werden.

Die Beleuchtungseinrichtung der erfindungsgemäßen Vorrichtung weist vorzugsweise mindestens eine Blitzlichtquelle auf, die sichtbares Licht abgibt.

Nach einer weiteren bevorzugten Ausgestaltung umfasst die Beleuchtungseinrichtung mindestens eine Infrarotlichtquelle, die Wärmestrahlen außerhalb des sichtbaren Farbspektrums aussendet. Die Bildaufnahmeeinrichtung ist dann dementsprechend aus einer infrarotlichtempfindlichen Kameraeinrichtung gebildet. Die Infrarotlichtquelle, bei es sich insbesondere um eine Infrarotblitzlichtquelle handeln kann, dient der Messung der Pupillenreaktion des Fahrers während der Fahrt. Neben der durch äußere Lichtquellen, insbesondere durch Licht entgegenkommender Fahrzeuge hervorgerufenen Pupillenreaktion können mit der infrarotlichtempfindlichen Kameraeinrichtung auch die Größe der Cornea-Oberfläche, die Lidschlagfrequenz, die Lidschlagdauer und/oder Pupillendurchmesserschwingungen wenigstens eines Auges des Fahrers erfasst werden. Anhand eines Vergleichs der entsprechenden Messwerte mit zugeordneten Normalwerten lässt sich eine einsetzende bzw. vorhandene Schläfrigkeit des Fahrers ermitteln. Eine ermittelte Schläfrigkeit kann wiederum durch akustische und/oder optische Signalgeber signalisiert werden, um der Schläfrigkeit des Fahrers entgegenzuwirken bzw. den Fahrer oder Dritte zu warnen.

Eine weitere vorteilhafte Ausgestaltung der erfindungsgemäßen Vorrichtung besteht in diesem Zusammenhang darin, dass die Auswerteeinrichtung in Abhängigkeit einer Veränderung der sichtbaren Größe der Cornea-Oberfläche, der Lidschlagfrequenz und/oder der Lidschlagdauer des Auges und/oder dem Auftreten von Pupillendurchmesserschwingungen eine auf das Gesichtsfeld des Fahrers gerichtete oder ausrichtbare Beleuchtungseinrichtung ansteuert, die ein einer Ermüdung des Fahrers entgegenwirkendes diffuses, breitflächiges.Licht aussendet.

Eine andere vorteilhafte Ausgestaltung der erfindungsgemäßen Vorrichtung ist **dadurch gekennzeichnet, dass** die Bildaufnahmeeinrichtung, die Auswerteeinrichtung und/oder der Datenspeicher mit mindestens einer Schnittstelle für eine Signal- und/oder Datenübertragung versehen sind. Die Schnittstelle ermöglicht eine einfache Datenübertragung von und zu anderen mobilen oder zentralen Überwachungs- und/oder Datenverarbeitungsgeräten. Die Schnittstelle kann dabei insbesondere als Sende-Empfänger-Einheit für eine drahtlose Signal- bzw. Datenübertragung ausgebildet sein.

Nach einer weiteren besonders bevorzugten Ausgestaltung sind die Beleuchtungseinrichtung und/oder die Bildaufnahmeeinrichtung in einer dem Fahrer zugeordneten Sonnenblende des Fahrzeuges integriert.

Um einer Manipulation der erfindungsgemäßen Vorrichtung vorzubeugen, sieht eine weitere Ausgestaltung vor, dass bei einer Funktionsstörung der Bildaufnahmeeinrichtung und/oder einer Funktionsstörung der Beleuchtungseinrichtung und/oder einer Funktionsstörung des ein akustisches und/oder optisches Warnsignal abgebenden Signalgebers die Auswerteeinrichtung auf mindestens eine Steuerungseinrichtung derart einwirkt, dass eine Inbetriebnahme des Fahrzeuges oder eine Weiterfahrt nach einem Halt des im Betriebszustand befindlichen Fahrzeuges verhindert wird.

Nachfolgend wird die Erfindung anhand einer mehrere Ausführungsbeispiele darstellenden Zeichnung näher erläutert. Es zeigen:
- Fig. 1: eine Blockdiagrammdarstellung einer erfindungsgemäßen Vorrichtung zur Erfassung der Fahrtüchtigkeit eines Fahrers in einem Fahrzeug;
- Fig. 2: eine schematische Darstellung eines Fahrers in Seitenansicht am Lenkrad eines Kraftfahrzeuges;
- Fig. 3: eine schematische Darstellung des Fahrers gemäß Fig. 2 in Draufsicht;
- Fig. 4: eine perspektivische Darstellung einer hochgeschwenkten Sonnenblende aus der Sicht des Fahrers;
- Fig. 5: eine perspektivische Darstellung einer Sonnenblende gemäß Fig. 4 von außen in Richtung des Fahrers betrachtet;
- Fig. 6: eine weitere perspektivische Darstellung der Sonnenblende gemäß Fig. 4;
- Fig. 7: eine Querschnittdarstellung der Sonnenblende gemäß Fig. 4 in einer hochgeschwenkten Stellung sowie in einer heruntergeschwenkten Stellung; und
- Fig. 8: eine weitere perspektivische Darstellung der Sonnenblende gemäß Fig. 4 in einer heruntergeschwenkten Stellung aus der Sicht des Fahrers.

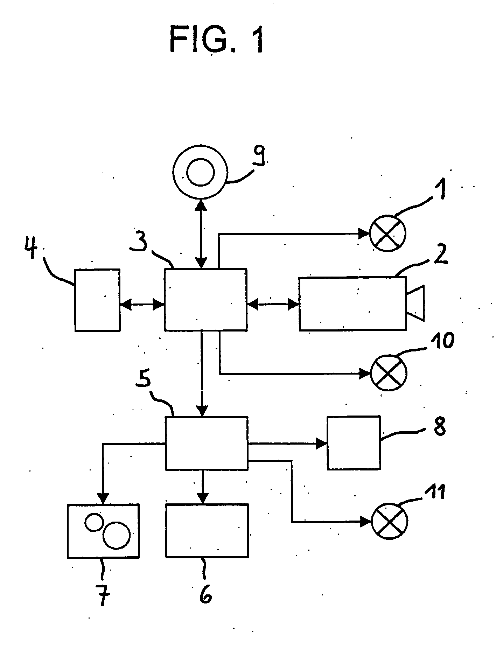

Die in Fig. 1 schematisch dargestellte Vorrichtung weist eine Beleuchtungseinrichtung 1 auf, mit der wenigstens eine Auge, vorzugsweise beide Augen eines Fahrers eines Kraftfahrzeuges beleuchtet werden, wenn dieser auf dem Fahrersitz Platz genommen hat. Die Belegung des Fahrersitzes wird durch einen oder mehrere geeignete Sensor, beispielsweise einen Drucksensor (nicht gezeigt) und/oder einen optischen Sensor, insbesondere eine Kamera erfasst. Die Beleuchtungseinrichtung 1 besteht aus einer Blitzleuchte, die sichtbares Licht abstrahlt, oder einer Leuchte, die kurzzeitig intermittierend sichtbares Licht abstrahlt. Die Intensität bzw. Helligkeit des abgestrahlten Lichtes ist dabei eine Funktion der Zeit. Der Pupillendurchmesser des beleuchteten Auges ändert sich mit dem abgestrahlten Licht bzw. dessen Helligkeit.

Der Beleuchtungseinrichtung 1 ist eine Bildaufnahmeeinrichtung 2 zugeordnet, die aus einer Kamera besteht, mit der Bilder des bzw. der beleuchteten Augen des Fahrers aufgenommen werden. Die aufgenommenen Bilder werden mit einer Auswerteeinrichtung 3 ausgewertet, die mit einem Datenspeicher 4 versehen ist.

Die Auswerteeinrichtung 3 vergleicht dabei aus den aufgenommenen Bildern gewonnene Messwerte der Pupillenreaktion des Fahrers mit Normalwerten für die Pupillenreaktion, die im Datenspeicher 4 gespeichert sind. Der Vergleich bezieht sich insbesondere auf die Pupillenreaktionszeit. Da die Pupillenreaktionszeit bei verschiedenen, fahrtüchtigen Personen unterschiedliche Werte annehmen kann und somit in einer gewissen Bandbreite liegt, ist im Datenspeicher 4 vorzugsweise ein bestimmter Grenzwert oder Soll-Wert für die bei Fahrtüchtigkeit übliche Pupillenreaktionszeit gespeichert.

Unterschreitet die ermittelte Pupillenreaktionszeit den Normalwert (Soll-Wert) bzw. Grenzwert (Mindestwert), so wirkt die Auswerteeinrichtung 2 auf eine Steuerungseinrichtung 5 derart ein, dass eine Inbetriebnahme des Kraftfahrzeuges oder eine Weiterfahrt nach einem Halt des im Betriebszustand befindlichen Kraftfahrzeuges verhindert wird.

In dem dargestellten Ausführungsbeispiel steht die Steuerungseinrichtung 5 in Wirkverbindung mit der Zündanlage 6, beispielsweise mit dem Zündschloßschalter und/oder dem Anlasser, so dass gegebenenfalls der Motorstart gesperrt wird. Darüber hinaus ist in Fig. 1 auch die Möglichkeit angedeutet, dass mittels der Steuerungseinrichtung ein Einlegen zumindest von Vorwärtsgängen des Schalt- oder Automatikgetriebes 7 des Fahrzeuges sperrbar ist.

Die Bildaufnahmeeinrichtung 2, die Auswerteeinrichtung 3, der Datenspeicher 4 und/oder die Steuerungseinrichtung 5 sind vorzugsweise mit mindestens einer Schnittstelle versehen, über die eine externe Signal- und/oder Datenübertragung erfolgen kann.

An der Auswerteeinrichtung 3 bzw. der ihr zugeordneten Steuerungseinrichtung 5 ist ferner ein Signalgeber 8 angeschlossen, der bei entsprechender Ansteuerung ein akustisches und/oder optisches Warnsignal abgibt. Der Signalgeber 8 wird über die Steuerungseinrichtung 5 von der Auswerteeinrichtung 3 angesteuert, wenn diese eine Unterschreitung der gespeicherten "normalen" Pupillenreaktionszeit durch die für den Fahrer tatsächlich gemessene Pupillenreaktionszeit ermittelt.

An der Auswerteeinrichtung 3 ist des weiteren ein Fingerabdrucksensor 9 angeschlossen, der zur Identifikation des Fahrers dient. Die biometrische Daten eines mit dem Sensor erfassten Fingerabdruckes sind in dem Datenspeicher 4 speicherbar. Die Auswerteeinrichtung 3 vergleicht den erfassten Fingerabdruck mit den Daten eines oder mehrerer gespeicherter Fingerabdrücke. Bei Nichtübereinstimmung der verglichenen Daten innerhalb vorgegebener Toleranzgrenzen wirkt die Auswerteeinrichtung 3 auf die Steuerungseinrichtung 5 ein, die dann eine Inbetriebnahme des Fahrzeuges bzw. gegebenenfalls nach einem Halt des fahrenden Fahrzeuges eine Weiterfahrt verhindert. Die Inbetriebnahme bzw. Weiterfahrt des Fahrzeuges wird in diesem Fall nur freigegeben, wenn die Auswerteeinrichtung 3 durch Prüfung der Pupillenreaktionszeit die Fahrtüchtigkeit des hinsichtlich seines Fingerabdruckes "unbekannten" Fahrers positiv festgestellt hat.

Alternativ oder ergänzend können in dem Datenspeicher 4 auch andere biometrische Daten einer oder mehrerer Personen gespeichert werden. Vorzugsweise werden biometrische Daten wie Irisstrukturkennzeichen, Augenfarbe, Augenabstand, Augenfläche, Nasengröße, Mundgröße und/oder Gesichtsform des jeweiligen Fahrers mit der Bildaufnahmeeinrichtung erfasst und in dem Datenspeicher 4 gespeichert. Die Auswerteeinrichtung 3 vergleicht dann zur Identifikation des Fahrers wiederum die erfassten biometrischen Daten mit den gespeicherten biometrischen Daten. Bei Nichtübereinstimmung der verglichenen Daten innerhalb vorgegebener Toleranzgrenzen wird ein entsprechendes Signal an die Steuerungseinrichtung 5 abgegeben, die darauf die Inbetriebnahme bzw. nach einem Halt des im Betriebszustand befindlichen Fahrzeuges eine Weiterfahrt blockiert. Auch in diesem Fall wird die Inbetriebnahme bzw. Weiterfahrt des Fahrzeuges nur dann freigegeben, wenn die Auswerteeinrichtung 3 durch Prüfung der Pupillenreaktionszeit die Fahrtüchtigkeit des hinsichtlich seiner erfassten biometrischen Daten "unbekannten" Fahrers positiv festgestellt hat.

Die in Fig. 1 dargestellte Vorrichtung weist ferner eine auf das Gesichtsfeld des Fahrers ausgerichtete bzw. ausrichtbare Infrarotlichtquelle 10 auf. Die Bildaufnahmeeinrichtung 2 ist dementsprechend aus einer infrarotlichtempfindlichen Kameraeinrichtung gebildet oder umfasst eine solche Kameraeinrichtung.

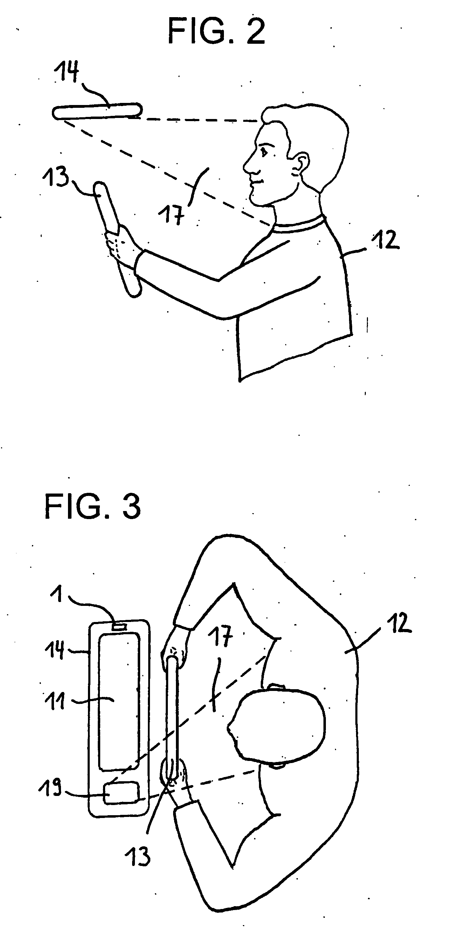

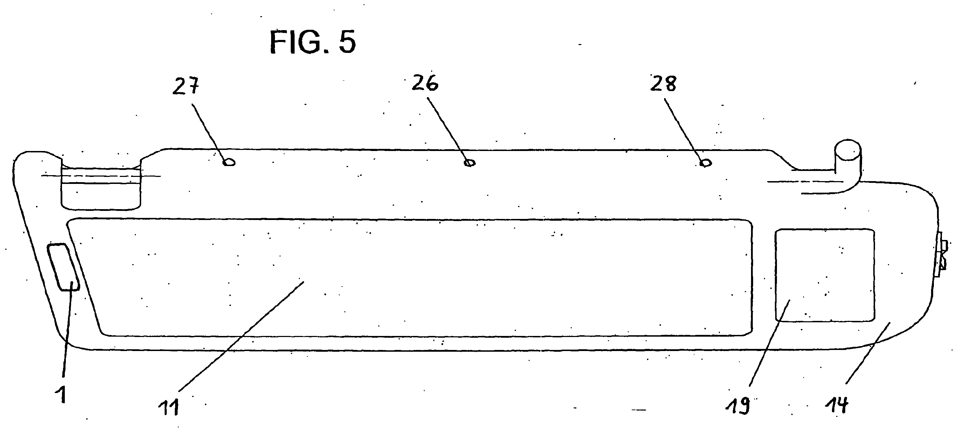

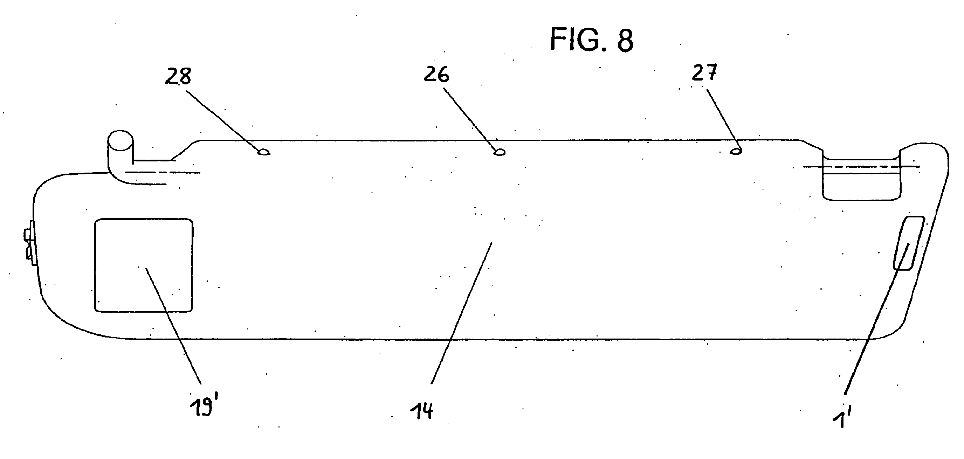

Die Auswerteeinrichtung 3 ist vorzugsweise so ausgebildet, dass sie in Abhängigkeit einer Veränderung der sichtbaren Größe der Cornea-Oberfläche, der Lidschlagfrequenz und/oder der Lidschlagdauer des bzw. der mit der Kameraeinrichtung 2 erfassten Augen des Fahrers und/oder beim Auftreten von Pupillendurchmesserschwingungen eine auf das Gesichtsfeld des Fahrers gerichtete oder ausrichtbare Beleuchtungseinrichtung 11 ansteuert, die ein einer Ermüdung des Fahrers entgegenwirkendes diffuses, breitflächiges Licht aussendet. Dies wird unter Bezug auf die Figuren 2 bis 8 noch näher beschrieben.

Die Beleuchtungseinrichtungen 1, 10 und 11 sowie die Bildaufnahmeeinrichtung 2 der erfindungsgemäßen Vorrichtung können vorzugsweise in einer dem Fahrer zugeordneten Sonnenblende integriert werden.

Die Figuren 2 und 3 zeigen einen Fahrer 12 am Lenkrad 13 eines Kraftfahrzeuges. Dem Fahrer ist wie üblich eine verschwenkbare Sonnenblende 14 zugeordnet, wobei Fig. 2 die Situation zeigt, in welcher sich die Sonnenblende 14 in ihrer eingeklappten Stellung befindet. Diese Situation entspricht in der Regel der Situation bei einer Nachtfahrt oder einer längeren Tunnelfahrt.

Die der Erfassung der Fahrtüchtigkeit dienende Beleuchtungseinrichtung 1, mit der wenigstens ein Auge des Fahrers 12 blitzlichtartig oder intermittierend beleuchtet wird, ist doppelt ausgeführt und auf beiden Seiten der Sonnenblende 14 angeordnet. Vorzugsweise wird von den beiden Beleuchtungseinrichtungen 1, 1' nur die Beleuchtungseinrichtung eingeschaltet, die je nach Stellung der Sonnenblende 14 dem Gesichtsfeld des Fahrers 12 zugewandt ist.

Am vorderen Rand der Sonnenblende 14, der in der eingeklappten Stellung der Sonnenblende 14 dem Fahrer 12 zugewandt ist, ist ein Lichtsensor 15 angeordnet, mit dem die Helligkeit im Bereich des Gesichtsfeldes des Fahrers 12 erfasst wird.

Die Sonnenblende 14 ist weiter mit einer eine Infrarotbeleuchtung umfassenden Bildaufnahmeeinrichtung zum Aufnehmen von Bildern des Bereiches wenigstens eines Auges des Fahrers versehen. Der Bildaufnahmeeinrichtung 2 ist eine Auswerteeinrichtung 3 zugeordnet, die von der Bildaufnahmeeinrichtung 2 aufgenommene Bilder auswertet und in Abhängigkeit einer Veränderung der sichtbaren Größe der Cornea-Oberfläche, der Lidschlagfrequenz und/oder der Lidschlagdauer des Auges die auf das Gesichtsfeld des Fahrers 12 gerichtete bzw. ausrichtbare Beleuchtungseinrichtung 11 ansteuert, die breitflächig ein einer Ermüdung des Fahrers entgegenwirkendes Licht emittiert.

Die Feststellung eines kritischen Müdigkeitszustandes des Fahrers 12 kann insbesondere auf der sogenannten Cornea-Reflexions-Methode basieren, bei der der Reflexionsgrad der Hornhaut (Cornea), welcher vom Öffnungsgrad des Augenlides abhängt, gemessen wird.

Die Auswerteeinrichtung 3 kann durch einen geeigneten Rechner (Bordcomputer) bzw. Mikroprozessor mit Speichereinrichtung realisiert sein. Vorzugsweise kann auch die Auswerteeinrichtung 3 in der Sonnenblende 14 integriert sein. Sie kann jedoch auch an einem anderen Ort im Fahrzeug angeordnet werden. Der Rechner bzw. Mikroprozessor steht dann über einen Daten- und Spannungsversorgungsanschluss mit den elektrooptischen und elektronischen Komponenten der Sonnenblende 14 im Dialog.

Die Auswerteeinrichtung 3 ist ferner mit einer Warnsignaleinrichtung (in den Figuren 2 bis 8 nicht dargestellt) verbunden, die den Fahrer durch ein optisches und/oder akustisches Warnsignal warnt, wenn die Auswerteeinrichtung 3 bei ihm einen kritischen Müdigkeitszustandes festgestellt hat.

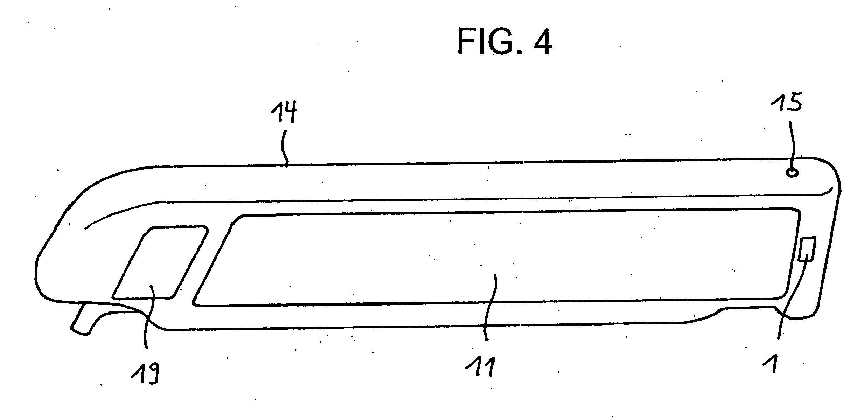

Die Bildaufnahmeeinrichtung 2 umfasst eine Miniatur-Kamera 16, die ein lichtempfindliches CCD-Array, eine Linse, eine Blende und eine Fokussiereinrichtung aufweist. Die Kamera 16, die für den Fahrer unsichtbar in der Sonnenblende 14 angeordnet ist, erfasst das Gesichtsfeld 17 des Fahrers über eine Umlenkoptik 18 in Form eines Spiegels, der vorzugsweise konvex ausgebildet ist. Im Strahlengang S der Kamera 16 ist ferner ein Teilerspiegel 19 angeordnet, der eine Öffnung innerhalb der Wandung bzw. Hülle der Sonnenblende 14 abdeckt und für den Fahrer 12 sichtbar ist (vgl. Figuren 3 und 4).

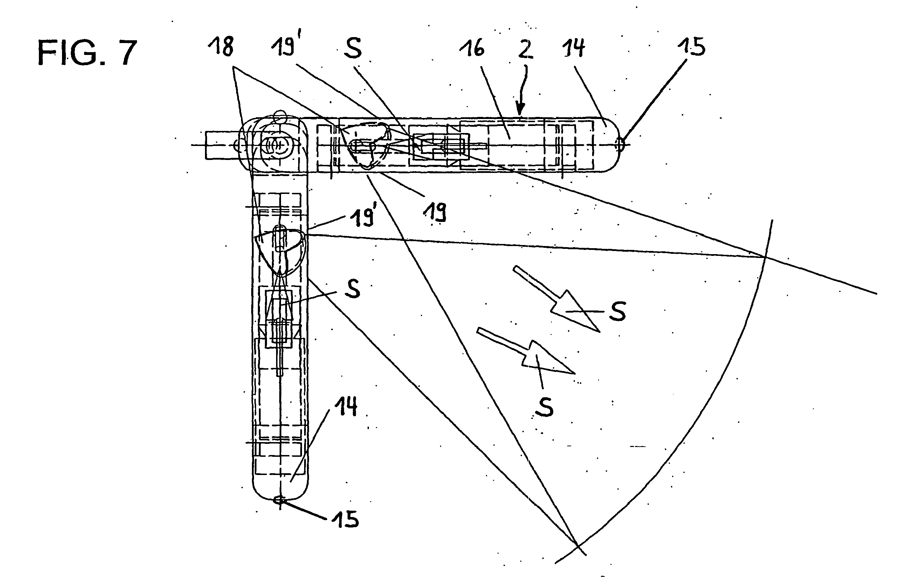

Ein zweiter entsprechender Teilerspiegel 19' ist auf der gegenüberliegenden Seite der Sonnenblende 14 angeordnet und für den Fahrer ebenfalls sichtbar, wenn die Sonnenblende 14 ausgeklappt wird. Wie insbesondere in Fig. 7 zu erkennen ist, sind die beiden Teilerspiegel 19, 19' so angeordnet, dass die Kamera 16 in der eingeklappten Stellung der Sonnenblende 14 über den Teilerspiegel 19 und in der ausgeklappten Stellung über den Teilerspiegel 19' Bilder des Bereichs wenigstens eines Auges des Fahrers aufnimmt.

Der Umlenkspiegel 18 ist beweglich gelagert. Die räumliche Lage des Umlenkspiegels 18 wird mittels eines einen Motor 20 und ein Getriebe 21 aufweisenden Stellantriebes in Abhängigkeit der Sitzposition bzw. der Größe des Fahrers automatisch eingestellt. Der Motor 20 des Stellantriebes wird hierzu von der Auswerteeinrichtung angesteuert. In dem der Auswerteeinrichtung zugeordneten Datenspeicher sind ein oder mehrere Referenzbilder gespeichert, die einem zu erfassenden Gesichtsfeld (Gesichtsmuster) entsprechen. Die Auswerteeinrichtung 3 vergleicht die von der Kamera 16 aufgenommenen Bilder mit den Referenzbildern und verstellt bei einer einen bestimmten Grenzwert übersteigenden Abweichung zwischen aufgenommenen Bildern und Referenzbild den Umlenkspiegel 18 so weit, bis die aufgenommenen Bilder mit dem bzw. den Referenzbildern wieder in einem bestimmten Maß übereinstimmen.

Die Beleuchtungseinrichtung 11 der erfindungsgemäßen Vorrichtung besteht vorzugsweise aus einem plattenförmigen, im wesentlichen rechteckigen Lichtleiter, der eine breitflächige Lichtaustrittsfläche aufweist und diffuses Licht abstrahlt. Der Lichtleiter besteht aus Glas oder einem glasartigen Kunststoff, beispielsweise Acrylglas. An der der Lichtaustrittsfläche gegenüberliegenden Seite weist der Lichtleiter vorzugsweise eine reflektierende Beschichtung oder Bedruckung auf. Bei dem dargestellten Ausführungsbeispiel nimmt die Lichtaustrittsfläche über die Hälfte einer Flachseite der Sonnenblende 14 ein. Sie ist nur auf der Seite der Sonnenblende 14 angeordnet, die in der ausgeklappten Stellung der Sonnenblende 14 in Fahrtrichtung weist.

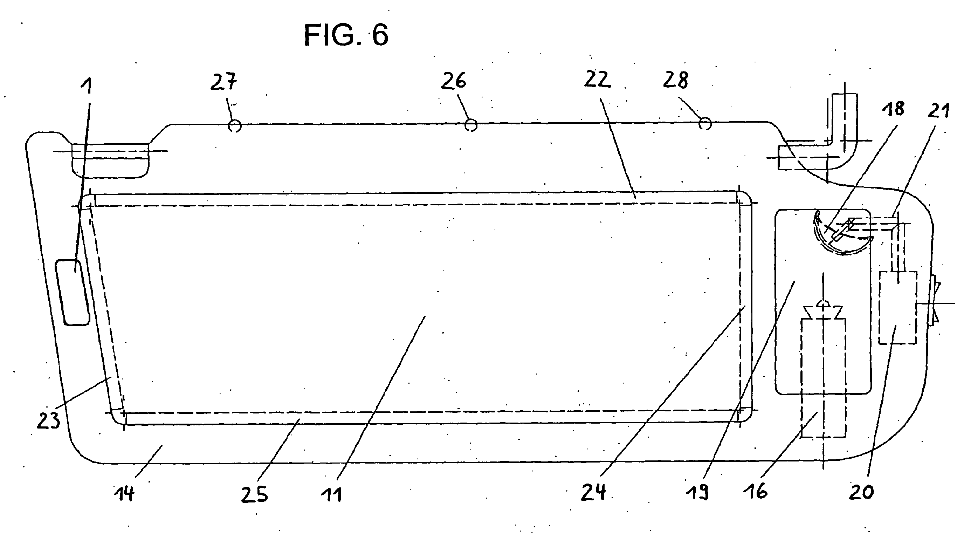

An den Rändern des Lichtleiters sind unterschiedliche Lichtquellen angeordnet, wobei ihr Licht jeweils über die schmalen Randflächen in den Lichtleiter eingekoppelt wird (vgl. Fig. 6). Am oberen Längsrand des Lichtleiters ist eine Leuchtdiodenleiste 22 angeordnet, mittels der sichtbares Licht in den Lichtleiter eingekoppelt wird. An den beiden Querrändern ist jeweils eine Infrarotlicht-Leuchtdiodenleiste 23 bzw. 24 angeordnet. Am unteren Längsrand befindet sich eine Leuchtdiodenleiste 25, die UV-Licht, und zwar UV-A-Strahlung im Bereich von 320 bis 400 nm, und/oder Licht in einem Spektrum von vorzugsweise 430 bis 620 nm abstrahlt. Versuche haben gezeigt, dass eine Lichtstrahlung mit diesem Spektrum einer Ermüdung von Testpersonen wirksam entgegenwirkt.

Der obere Rand der Sonnenblende 14 weist eine Öffnung auf, in die ein Lichtsensor 26 eingelassen ist. Der Lichtsensor 26 dient der Erfassung der äußeren Helligkeit und ist an einer elektronischen Steuerung angeschlossen, mittels der die Intensität der von der Beleuchtungseinrichtung 11 emittierten sichtbaren und unsichtbaren Lichtstrahlung in Abhängigkeit von der auf den Lichtsensor 26 fallenden Lichtstrahlung der Umgebung gesteuert wird. Vorzugsweise umfasst der Rechner bzw. Mikroprozessor der Auswerteeinrichtung 3 diese elektronische Steuerung.

Neben dem Lichtsensor 26 sind am oberen Rand der Sonnenblende 14 rechts und links zwei weitere Lichtsensoren 27, 28 angeordnet. Die Lichtsensoren 27, 28 dienen der Erfassung der Lichtstrahlung entgegenkommender Fahrzeuge bzw. äußerer Blendungsquellen während Nachtfahrten sowie längeren Tunnelfahrten. Die Lichtsensoren 27, 28 sind mit einer elektronischen Steuerung verbunden, welche zumindest die von der Beleuchtungseinrichtung 11 emittierte sichtbare Lichtstrahlung in Abhängigkeit von der auf die Lichtsensoren 27, 28 fallenden Lichtstrahlung der Blendungsquelle steuert. Zusätzlich kann aber auch die emittierte UV-Strahlung in Abhängigkeit von der erfassten Blendungsstrahlung gesteuert werden. Vorzugsweise übernimmt der Rechner bzw. Mikroprozessor der Auswerteeinrichtung 3 auch diese Steuerung.

In der ausgeklappten Stellung der Sonnenblende 14 ist die Lichtaustrittsfläche nicht dem Gesichtsfeld 17 des Fahrer zugewandt. Dies ist allerdings auch nicht zweckmäßig, da die Sonnenblende 14 in der Regel nur bei Fahrten am Tag ausgeklappt wird, nämlich dann, wenn die Sonne den Fahrer blendet. Auch in der ausgeklappten Stellung kann die Kamera 16 jedoch über den Teilerspiegel 19' das Gesichtsfeld 17 des Fahrers erfassen. Somit ist in der ausgeklappten Stellung der Sonnenblende 14 gewährleistet, dass in Abhängigkeit eines durch die Auswerteeinrichtung festgestellten Müdigkeitszustandes des Fahrers ein Warnsignal an ihn durch eine geeignete optische und/oder akustische Signaleinrichtung abgegeben wird.

Die vorstehend beschriebene Sonnenblende 14 ist somit eine multifunktionale Sonnenblende, die neben ihrer üblichen Funktion zusätzlich die Funktionen:
- der Erfassung der Fahrtauglichkeit eines Fahrers,
- der Überwachung der Wachsamkeit eines Fahrers,
- der automatischen Ausstrahlung eines einer Ermüdung des Fahrzeugführers entgegenwirkendes Lichtes und
- der Verhinderung einer Blendung des Fahrzeugführers durch das Fahrtlicht entgegenkommender Fahrzeuge bei Fahrten im Dunkeln übernimmt.

## Patentansprüche

1. Vorrichtung zur Erfassung der Fahrtüchtigkeit eines Fahrers in einem Fahrzeug, mit einer Beleuchtungseinrichtung (1) zum Beleuchten wenigstens eines Auges des Fahrers (12), einer Bildaufnahmeeinrichtung (2) zum Aufnehmen von Bildern des beleuchteten Auges, einer Auswerteeinrichtung (3), die dem Auswerten der von der Bildaufnahmeeinrichtung (2) aufgenommenen Bilder dient, und einem Datenspeicher (4), wobei die Beleuchtungseinrichtung (1) wenigstens ein Auge des Fahrers (12) blitzlichtartig oder intermittierend beleuchtet, wobei die Auswerteeinrichtung (3) mittels der Bildaufnahmeeinrichtung (2) erfasste Messwerte der Pupillenreaktion des Fahrers mit mindestens einem im Datenspeicher (4) gespeicherten Normalwert einer Pupillenreaktion vergleicht und bei einer Unterschreitung des Normalwertes durch die Messwerte der Pupillenreaktion auf eine Steuerungseinrichtung (5) derart einwirkt, dass eine Inbetriebnahme des Fahrzeuges oder eine Weiterfahrt nach einem Halt des im Betriebszustand befindlichen Fahrzeuges verhindert wird, **dadurch gekennzeichnet, dass** in dem Datenspeicher (4) biometrische Daten mindestens eines Fingerabdruckes speicherbar und mittels eines Sensors (9) biometrische Daten eines Fingerabdruckes des jeweiligen Fahrers erfassbar und/oder in dem Datenspeicher (4) biometrische Daten mindestens einer Person bezüglich deren Irisstruktur, Augenfarbe, Augenabstand, Augenfläche, Nasengröße, Mundgröße und/oder Gesichtsform speicherbar und mittels der Bildaufnahmeeinrichtung (2) entsprechende biometrische Daten des jeweiligen Fahrers erfassbar sind, wobei die Auswerteeinrichtung (3) zu einer Identifikation des Fahrers die erfassten biometrischen Daten mit den gespeicherten biometrischen Daten vergleicht und bei Nichtübereinstimmung der verglichenen Daten innerhalb vorgegebener Toleranzgrenzen auf mindestens eine Steuerungseinrichtung (5) derart einwirkt, dass eine Inbetriebnahme des Fahrzeugs oder eine Weiterfahrt nach einem Halt des im Betriebszustand befindlichen Fahrzeugs verhindert wird.

2. Vorrichtung nach Anspruch 1,
**dadurch gekennzeichnet, dass**
mittels der Steuerungseinrichtung (5) ein Motorstart verhinderbar ist.

3. Vorrichtung nach Anspruch 1 oder 2,
**dadurch gekennzeichnet, dass**
mittels der Steuerungseinrichtung (5) ein Einlegen zumindest von Vorwärtsgängen des Schalt- oder Automatikgetriebes (7) des Fahrzeuges sperrbar ist.

4. Vorrichtung nach einem der Ansprüche 1 bis 3,
**dadurch gekennzeichnet, dass**
die Auswerteeinrichtung (3) bei einer Unterschreitung des gespeicherten Normalwertes einer Pupillenreaktion durch die erfassten Messwerte der Pupillenreaktion einen Signalgeber (8) ansteuert, der ein akustisches und/oder optisches Warnsignal abgibt.

5. Vorrichtung nach einem der Ansprüche 1 bis 4,
**dadurch gekennzeichnet, dass**
die Beleuchtungseinrichtung (1) mindestens eine Blitzlichtquelle aufweist.

6. Vorrichtung nach einem der Ansprüche 1 bis 5,
**dadurch gekennzeichnet, dass**
die Beleuchtungseinrichtung mindestens eine InfrarotLichtquelle (10) aufweist, die Wärmestrahlen außerhalb des sichtbaren Farbspektrums aussendet, wobei die Bildaufnahmeeinrichtung (2) aus einer infrarotempfindlichen Kameraeinrichtung (16) gebildet ist.

7. Vorrichtung nach einem der Ansprüche 1 bis 6,
**dadurch gekennzeichnet, dass**
die Bildaufnahmeeinrichtung (2), die Auswerteeinrichtung (3) und/oder der Datenspeicher (4) mit mindestens einer Schnittstelle für eine Signal- und/oder Datenübertragung versehen sind.

8. Vorrichtung nach einem der Ansprüche 1 bis 7,
**dadurch gekennzeichnet, dass** die Beleuchtungseinrichtung (1) und/oder die Bildaufnahmeeinrichtung (2) in einer dem Fahrer (12) zugeordneten Sonnenblende (14) des Fahrzeuges integriert sind.

9. Vorrichtung nach einem der Ansprüche 1 bis 8,
**dadurch gekennzeichnet, dass**
die Auswerteeinrichtung (3) in Abhängigkeit einer Veränderung der sichtbaren Größe der Cornea-Oberfläche, der Lidschlagfrequenz und/oder der Lidschlagdauer des Auges und/oder dem Auftreten von Pupillendurchmesserschwingungen eine auf das Gesichtsfeld des Fahrers gerichtete oder ausrichtbare Beleuchtungseinrichtung (11) ansteuert, die ein einer Ermüdung des Fahrers entgegenwirkendes diffuses, breitflächiges Licht aussendet.

10. Vorrichtung nach einem der Ansprüche 1 bis 9,
**dadurch gekennzeichnet, dass** die Auswerteeinrichtung (3) bei einer Funktionsstörung der Bildaufnahmeeinrichtung (2) und/oder einer Funktionsstörung der Beleuchtungseinrichtung (1) und/oder einer Funktionsstörung des ein akustisches und/oder optisches Warnsignal abgebenden Signalgebers (8) auf mindestens eine Steuerungseinrichtung (5) derart einwirkt, dass eine Inbetriebnahme des Fahrzeuges oder eine Weiterfahrt nach einem Halt des im Betriebszustand befindlichen Fahrzeuges verhindert wird.

## Claims

1. A device for determining the driving capability of a driver in a vehicle with an illumination device (1) for illuminating at least one of the driver's (12) eyes, a picture taking device (2) for taking pictures of the illuminated eye, an evaluation device (3) which serves to evaluate the pictures taken by the picture taking device (2), and a data storage (4), the illumination device (1) illuminating with flash type light or intermittently at least one of the driver's (12) eyes, the evaluation device (3) comparing the measured values taken for the driver's pupil reaction by means of the picture taking device (2) with a normal value for a pupil reaction stored in the data storage (4), and when the normal value is not reached by the measured values for the pupil reaction, having an effect upon a control device (5) such that the vehicle is prevented from starting up, or the vehicle in operational state is prevented from being driven on after it has stopped,
**characterised in that** biometric data of at least one finger print can be stored in the data storage (4) and biometric data of a finger print of the driver in question can be determined by means of a sensor (9), and/or biometric data for at least one person relating to their iris structure, eye colour, distance between the eyes, eye area, nose size, mouth size and/or face shape can be stored in the data storage (4) and corresponding biometric data of the driver in question can be determined by the picture taking device (2), the evaluation device (3) for identifying the driver comparing the biometric data established with the stored biometric data, and if the data compared do not correspond within pre-specified tolerance limits having an effect upon at least one control device (5) such that the vehicle is prevented from starting up, or the vehicle in operating state is prevented from being driven on after it has stopped.

2. The device according to Claim 1,
**characterised in that** an engine start up can be prevented by means of the control device (5).

3. The device according to Claims 1 or 2,
**characterised in that** by means of the control device (5) engagement of at least the forward gears of the manual or automatic transmission (7) of the vehicle can be blocked.

4. The device according to any of Claims 1 to 3,
**characterised in that** when the measured values for the pupil reaction fail to reach the normal value for a pupil reaction stored, the evaluation device (3) actuates a signal transmitter (8) which emits an acoustic and/or optical warning signal.

5. The device according to any of Claims 1 to 4,
**characterised in that** the illumination device (1) has at least one flash light source.

6. The device according to any of Claims 1 to 5,
**characterised in that** the illumination device has at least one infra-red light source (10) which emits heat rays
outside of the visible colour spectrum, the picture taking device (2) being formed by a camera device (16) sensitive to infra-red.

7. The device according to any of Claims 1 to 6,
**characterised in that** the picture taking device (2), the evaluation device (3) and/or the data storage (4) are provided with at least one interface for signal and/or data transfer.

8. The device according to any of Claims 1 to 7,
**characterised in that** the illumination device (1) and/or the picture taking device (2) are integrated in a vehicle sun visor (14) provided for the driver (12).

9. The device according to any of Claims 1 to 8,
**characterised in that** the evaluation device (3) actuates an illumination device (11) aligned or alignable to the visual field of the driver and which emits a diffuse, wide area of light which counters the driver's tiredness dependent upon a change to the visible size of the cornea surface, the lid closure frequency and/or the lid closure duration of the eye and/or the occurrence of pupil diameter oscillations.

10. The device according to any of Claims 1 to 9,
**characterised in that** if there is a functional failure of the picture taking device (2) and/or a functional failure of the illumination device (1) and/or a functional failure of the signal transmitter (8) emitting an acoustic and/or optical warning signal, the evaluation device (3) has an effect upon at least one control device (5) such that the vehicle is prevented from starting up or a vehicle in operating state is prevented from being driven on after it has stopped.

## Revendications

1. Dispositif pour détecter la capacité de conduire d'un conducteur dans un véhicule, avec un dispositif d'éclairage (1) pour éclairer au moins un oeil du conducteur (12), avec un dispositif d'acquisition d'images (2) pour prendre des images de l'oeil éclairé, avec un dispositif d'évaluation (3) pour évaluer les images acquises par le dispositif d'acquisition d'images (2), et avec une mémoire de données (4), le dispositif d'éclairage (1) éclairant au moins un oeil du conducteur (12) par éclairs ou par intermittence, le dispositif d'évaluation (3) comparant les valeurs de mesure de la réaction de pupille du conducteur, saisies au moyen du dispositif d'acquisition d'images (2), avec au moins une valeur normale d'une réaction de pupille, qui est mémorisée dans la mémoire de données (4), et, lorsque les valeurs de mesure de la réaction de la pupille sont inférieures à la valeur normale, ledit dispositif d'évaluation agissant sur un dispositif de commande (5), de manière à empêcher une mise en marche du véhicule ou la poursuite du trajet, après un arrêt du véhicule en état de fonctionnement, **caractérisé en ce que** des données biométriques d'au moins une empreinte digitale peuvent être mémorisées et que des données biométriques d'une empreinte digitale du conducteur concerné peuvent être saisies au moyen d'un détecteur (9) et / ou que des données biométriques d'au moins une personne, relatives à la structure de son iris, à la couleur de ses yeux, à l'intervalle entre ses yeux, à la surface de ses yeux, à la grandeur de son nez, à la grandeur de sa bouche et / ou à la forme de son visage, peuvent être mémorisées dans la mémoire de données (4), et que des données biométriques correspondantes du conducteur concerné peuvent être saisies au moyen du dispositif d'acquisition d'images (2), le dispositif d'évaluation (3) comparant les données biométriques saisies avec les valeurs biométriques mémorisées, afin d'identifier le conducteur, et, quand les données comparées ne concordent pas dans une limite de tolérance prédéterminée, agit sur un dispositif de commande (5) de manière à empêcher une mise en marche du véhicule ou la poursuite du trajet, après un arrêt du véhicule en état de fonctionnement.

2. Dispositif selon la revendication 1, **caractérisé en ce qu'**un démarrage du moteur peut être empêché au moyen du dispositif de commande (5).

3. Dispositif selon revendication 1 ou 2, **caractérisé en ce que** l'enclenchement d'au moins des marches avant de la boîte de vitesses manuelle ou de la boîte de vitesses automatique (7) du véhicule puisse être bloqué au moyen du dispositif de commande (5).

4. Dispositif selon l'une des revendications 1 à 3, **caractérisé en ce que** le dispositif d'évaluation (3), lorsque la valeur normale d'une réaction de pupille mémorisée est dépassée vers le bas par les valeurs de mesure de réaction de la pupille saisies, excite un poste transmetteur de signaux (8), qui lance un signal avertisseur acoustique et / ou optique.

5. Dispositif selon l'une des revendications 1 à 4, **caractérisé en ce que** le dispositif d'éclairage (1) présente au moins un flash.

6. Dispositif selon l'une des revendications 1 à 5, **caractérisé en ce que** le dispositif d'éclairage présente au moins une source de lumière infrarouge (10), qui émet un rayonnement thermique en dehors du spectre chromatique visible, le dispositif d'acquisition d'images (2) étant formé par une caméra (16) sensible aux infrarouges.

7. Dispositif selon l'une des revendications 1 à 6, **caractérisé en ce que** le dispositif d'acquisition d'images (2), le dispositif d'évaluation (3) et / ou la mémoire de données (4) sont pourvus d'au moins une interface pour une transmission de signaux et / ou de données.

8. Dispositif selon l'une des revendications 1 à 7, **caractérisé en ce que** le dispositif d'éclairage (1) et / ou le dispositif d'acquisition d'images (2) sont intégrés dans un pare-soleil (14) du véhicule, qui est affecté au conducteur (12).

9. Dispositif selon l'une des revendications 1 à 8, **caractérisé en ce que** le dispositif d'évaluation (3), en fonction d'un changement de la grandeur visible de la surface de la cornée, de la fréquence du battement de la paupière et / ou de la durée du battement de la paupière de l'oeil et / ou de l'apparition de fluctuations du diamètre de la pupille, excite un dispositif d'éclairage (11), dirigé ou pouvant être dirigé dans le champ visuel du conducteur, lequel dispositif émet un large faisceau de lumière diffuse combattant une fatigue du conducteur.

10. Dispositif selon l'une des revendications 1 à 9, **caractérisé en ce que**, lors d'une perturbation du fonctionnement du dispositif d'acquisition d'images (2) et / ou d'une perturbation du fonctionnement du dispositif d'éclairage (1) et / ou d'une perturbation du fonctionnement du poste transmetteur de signaux (8), lançant un signal avertisseur acoustique et / ou optique, le dispositif d'évaluation (3) agit sur au moins un dispositif de commande (5) de manière à empêcher une mise en marche du véhicule automobile ou la poursuite du trajet, après un arrêt du véhicule en état de fonctionnement.
